# DEMANDE DE BREVET EUROPEEN

(11) **EP 0 760 231 A1**
(43) Date de publication de la demande: **05.03.1997**
(21) Numéro de dépôt: 96401767.7
(22) Date de dépôt: 12.08.1996
(51) Int. Cl.: A61B 17/74

(54) **Implant pour ostéosynthèse d'épiphyse fémorale supérieure**

(30) Priorité: 23.08.1995 FR 9510076
(71) Demandeur: BIOMAT, 91892 Saclay (FR)
(72) Inventeur: Lahille, Michel, 91430 Vauhallan (FR); Cottin, Philippe, 78470 Saint Remy les Chevreuse (FR); Maresca, Christian, 13014 Marseille (FR); Yamaguchi, Toshio, Kobe, Hyogo (JP)
(74) Mandataire: Cabinet Martinet & Lapoux

(57) **Abrégé**

Dans le corps (1 ; 3) d'un implant est ménagé un conduit longitudinal de revascularisation qui est borgne à l'extrémité distale du corps à implanter dans un fragment osseux, tel que trochanter (TR), et qui débouche à travers des orifices transversaux (13 ; 341) et à l'extrémité proximale du corps à implanter dans un autre fragment osseux, tel que tête fémorale (TE). A l'extrémité distale du corps, au moins un tronçon autotaraudeur (14, 15) immobilise le corps (1) dans le milieu osseux, ou bien un embout d'ancrage (4) est monté à coulissement sur le corps (5) et contient un moyen amortissant (5) propre à pousser en direction proximale l'embout (4) et en direction distale le corps (3) afin de maintenir comprimée la fracture (FR2). L'extrémité distale du corps peut être montée dans une partie cylindrique d'une plaque fixée sur la diaphyse fémorale.

## Description

La présente invention concerne un implant notamment pour ostéosynthèse d'épiphyse fémorale supérieure de manière à maintenir ensemble des fragments osseux séparés par une fracture localisée dans le col du fémur ou sous la tête du fémur.

Il est connu qu'un tel implant d'ostéosynthèse est sous la forme d'une plaque métallique d'ostéosynthèse, dite également clou-plaque ou vis-plaque présentant une courte branche supérieure et une longue branche inférieure séparées par un coude. La plaque épouse sensiblement le profil vertical de la face externe du fémur entre l'épiphyse supérieure et la diaphyse fémorale de manière à être fixée contre cette face. Une ou deux longues vis parallèles à extrémité autotaraudeuse traversent le trochanter et le col du fémur fracturé et sont vissées dans la tête de fémur. La tête généralement fraisée de ces vis est noyée dans une fraisure de la branche courte supérieure de la plaque appliquée contre la face externe de l'épiphyse. Entre trois à huit vis plus courtes sont vissées perpendiculairement dans la diaphyse fémorale pour maintenir la branche plate longue inférieure de la plaque contre la face externe de la diaphyse.

La fixation de la branche longue de la plaque à la diaphyse maintient immobile la branche courte contre laquelle s'appuie une longue vis afin d'unir le fragment de la tête fémorale contre le fragment du trochanter.

Les vis longues pleines déséquilibrent les pressions intracéphaliques dans les fragments osseux au centre du site de la nécrose où l'irrigation vasculaire a été supprimée à la suite de la fracture. En outre, la longueur de la plaque et le grand nombre de vis nécessitent un large champ opératoire et une opération longue.

La présente invention vise à fournir un implant du type longue vis permettant la revascularisation d'un foyer osseux fragmenté.

A cette fin, un implant pour ostéosynthèse d'une fracture entre des premier et second fragments osseux comprenant un corps longiligne ayant une portion autotaraudeuse située à l'une des d'extrémités proximale et distale du corps à implanter respectivement dans les premier et second fragments osseux, est caractérisé en ce que dans le corps sont prévus des orifices transversaux à travers lesquels débouche un conduit longitudinal ayant une extrémité distale borgne et une extrémité proximale débouchante.

Bien que l'implant selon l'invention soit utilisable pour réunir au moins deux fragments osseux de part et d'autre d'une fracture située particulièrement au niveau d'une épiphyse osseuse, il est destiné particulièrement à l'ostéosynthèse d'une épiphyse fémorale supérieure qui est le siège d'une fracture au niveau du col fémoral ou sous la tête épiphysaire fémorale. Dans ce dernier cas, le premier fragment osseux appartient à la tête fémorale et le second fragment osseux appartient au trochanter fémoral.

Selon une première réalisation, le conduit longitudinal de revascularisation est obstrué par un bouchon à l'extrémité distale du corps de l'implant. Ce bouchon sert à empêcher tout épanchement vasculaire depuis le conduit longitudinal vers la face externe épiphysaire. Le bouchon peut être vissé dans l'extrémité distale du conduit ; en particulier, l'extrémité distale du conduit comprend un taraudage dans lequel est vissée une tige filetée du bouchon.

De manière à visser le corps dans un trou perforé dans les fragments osseux, l'extrémité distale du conduit comprend un chambrage débouchant à l'extérieur du corps et ayant une section, de préférence polygonale, pour recevoir une extrémité à section complémentaire d'une clé.

Selon cette première réalisation, la portion autotaraudeuse est située à l'extrémité distale du corps afin que le corps soit ancré dans le second fragment osseux, tel que le trochanter. De préférence, la portion autotaraudeuse est composée de deux tronçons filetés ayant des filetages différents afin d'immobiliser en translation le corps dans le second fragment osseux après régénération osseuse autour du corps de l'implant. La différence de filetage entre les deux tronçons filetés peut être une différence entre leurs pas et/ou peut être une différence entre leurs diamètres.

Selon un exemple de réalisation préféré, la portion autotaraudeuse comprend un premier tronçon fileté proximal et un second tronçon fileté distal ayant au moins l'un de pas et diamètre nominal respectivement plus grand que l'un respectif de pas et diamètre nominal du premier tronçon fileté.

Selon une seconde réalisation, la portion autotaraudeuse est située à l'extrémité proximale du corps pour être implantée dans le premier fragment osseux, tel que tête fémorale. Cette portion autotaraudeuse, comme également dans la première réalisation, peut comporter à son extrémité distale au moins une encoche longitudinale d'amorce de taraudage.

Dans la seconde réalisation, il est prévu des moyens pour comprimer la fracture, c'est-à-dire pour resserrer entre eux les fragments osseux de manière à obtenir un montage à solidité maximale, sans faire appel à une assise, telle que plaque d'ostéosynthèse, s'étendant sur la face externe diaphysaire. Pour parvenir à cette compression, à l'extrémité distale du corps est prévu un embout monté à coulissement autour d'une portion intermédiaire du corps et contenant un moyen amortissant propre à pousser en direction proximale l'embout et en direction distale le corps. De cette manière, la portion autotaraudeuse ancrée dans le premier fragment osseux, tel que tête fémorale, est tirée en direction de la fracture et donc du second fragment osseux, tel que trochanter, et l'embout ancré dans le second fragment osseux est poussé suivant la direction opposée, également vers la fracture.

Le moyen amortissant est de préférence un ressort hélicoïdal qui peut être imprégné d'une matière plastique afin de freiner sa détente après implantation. Le ressort hélicoïdal est disposé dans l'embout de sorte qu'une extrémité proximale du ressort soit appliquée entre un épaulement interne à l'embout et un élément lié au corps soit placé contre l'extrémité distale du ressort. Cet élément peut être un bouchon fileté qui est vissé sur un tronçon fileté situé à l'extrémité distale du corps et qui est logé dans un conduit interne à l'embout.

Plus précisément, l'embout peut comprendre un conduit interne comportant au moins un chambrage proximal et un chambrage intermédiaire en direction distale. Le chambrage proximal peut avoir une section polygonale pour coulisser sur un tronçon à section polygonale du corps qui peut être situé sensiblement au niveau de l'extrémité distale du conduit longitudinal dans le corps. Le chambrage intermédiaire est de préférence plus large que le chambrage proximal pour recevoir le moyen amortissant, tel que le ressort précité, appliqué contre un épaulement entre les chambrages proximal et intermédiaire. Un élément logé dans l'embout est lié à l'extrémité distale du corps pour comprimer le moyen amortissant contre l'épaulement.

De manière à visser à la fois le corps et l'embout respectivement dans les premier et second fragments osseux, notamment dans la tête fémorale et le trochanter fémoral, le conduit interne dans l'embout comporte un chambrage distal, qui peut être plus large que le chambrage intermédiaire, ayant une section de préférence polygonale pour recevoir une extrémité complémentaire de clé de vissage.

D'une manière analogue à la portion autotaraudeuse située à l'extrémité distale du corps selon la première réalisation, la périphérie de l'embout selon cette seconde réalisation peut comprendre un premier tronçon autotaraudeur. Le pas du premier tronçon autotaraudeur est de préférence sensiblement égal à celui de la portion autotaraudeuse proximale du corps de manière à faciliter le vissage à la fois de l'embout distal et de la portion autotaraudeuse proximale du corps.

La périphérie de l'embout peut comprendre un second tronçon autotaraudeur ayant un filet différent de celui du premier tronçon autotaraudeur lorsqu'il est prévu, et/ou différent de celui de la portion autotaraudeuse située à l'extrémité du corps, c'est-à-dire ayant un pas et/ou un diamètre différents de ceux respectifs de ladite portion autotaraudeuse. Cette caractéristique interdit tout dévissage de l'embout et recul en direction distale de l'embout. Selon un exemple préféré, la périphérie de l'embout comprend à son extrémité proximale le premier tronçon autotaraudeur et à son extrémité distale le second tronçon autotaraudeur qui a un pas et/ou un diamètre nominal respectivement plus grand que le pas et le diamètre nominal du premier tronçon autotaraudeur.

Selon une troisième réalisation destinée à consolider des fractures sous-trochantériennes, l'implant comprend une plaque ayant une portion d'ancrage pour être fixée contre la diaphyse du second fragment osseux, et une portion cylindrique qui est traversée à glissement par l'embout et contre laquelle une embase distale de l'embout est appliquée. La portion cylindrique de la plaque est oblique par rapport à la portion d'ancrage de la plaque afin que le corps soit maintenu sensiblement perpendiculairement à la fracture.

L'implant peut comprendre un moyen situé entre l'embase distale de l'embout et la portion cylindrique de la plaque pour amortir des déplacements relatifs entre l'embout et la plaque, et ainsi atténuer des efforts de levier dans le foyer de fracture.

Le moyen de l'implant pour amortir peut comprendre une rondelle en matériau amortissant qui est de préférence en contact avec une ou deux rondelles métalliques.

De préférence, la plaque de l'implant a une face à appliquer contre la diaphyse du second fragment, qui est concave transversalement, et la portion cylindrique de la plaque de l'implant est tronquée et présente une base qui est inclinée par rapport à l'axe de la portion cylindrique et concave transversalement et sensiblement convexe longitudinalement. Ainsi la plaque épouse la face diaphysaire du fémur sous le trochanter et répartit les efforts de traction progressifs exercés sur l'embout tout en maintenant axialement l'embout.

D'autres caractéristiques et avantages de la présente invention apparaîtront plus clairement à la lecture de la description suivante de plusieurs réalisations préférées de l'invention en référence aux dessins annexés correspondants dans lesquels :
- la figure 1 est une vue en coupe axiale longitudinale d'un corps de premier implant selon l'invention ;
- la figure 2 est une vue longitudinale d'un bouchon du corps du premier implant ;
- la figure 3 est une vue en coupe schématique dans un plan médio-latéral d'une épiphyse supérieure de fémur dans laquelle sont percés un trou et deux chambrages pour implanter le premier implant ;
- la figure 4 est une vue en coupe schématique dans un plan médio-latéral d'une épiphyse supérieure de fémur montrant le vissage du premier implant ;
- la figure 5 est une vue analogue à la figure 4 montrant l'introduction du bouchon dans le premier implant ;
- la figure 6 est une vue longitudinale d'un corps d'un second implant selon l'invention ;
- la figure 7 est une vue éclatée en coupe axiale longitudinale du second implant ;
- la figure 8 est une vue en coupe schématique dans un plan médio-latéral d'une épiphyse supérieure de fémur montrant l'introduction d'un ressort et d'un bouchon dans le corps du second implant déjà introduit dans l'épiphyse ;
- la figure 9 est une vue en coupe schématique dans un plan médio-latéral d'une extrémité supérieure de fémur dans laquelle deux premiers implants et un second implant sont implantés ;
- la figure 10 est une vue en coupe schématique dans un plan médio-latéral d'une épiphyse supérieure de fémur dans laquelle un troisième implant à plaque diaphysaire selon l'invention est implanté ;
- la figure 11 est une vue de la plaque diaphysaire prise suivant la direction de la flèche F11 dans la figure 10 ; et
- la figure 12 et une vue en coupe axiale d'un embout du troisième implant.

Dans la description ci-après de plusieurs réalisations d'implant d'ostéosynthèse selon l'invention, les dimensions des différentes pièces composant les implants sont données à titre d'exemples non limitatifs. Les corps, bouchons et embouts des implants sont métalliques, par exemple en alliage à base de titane.

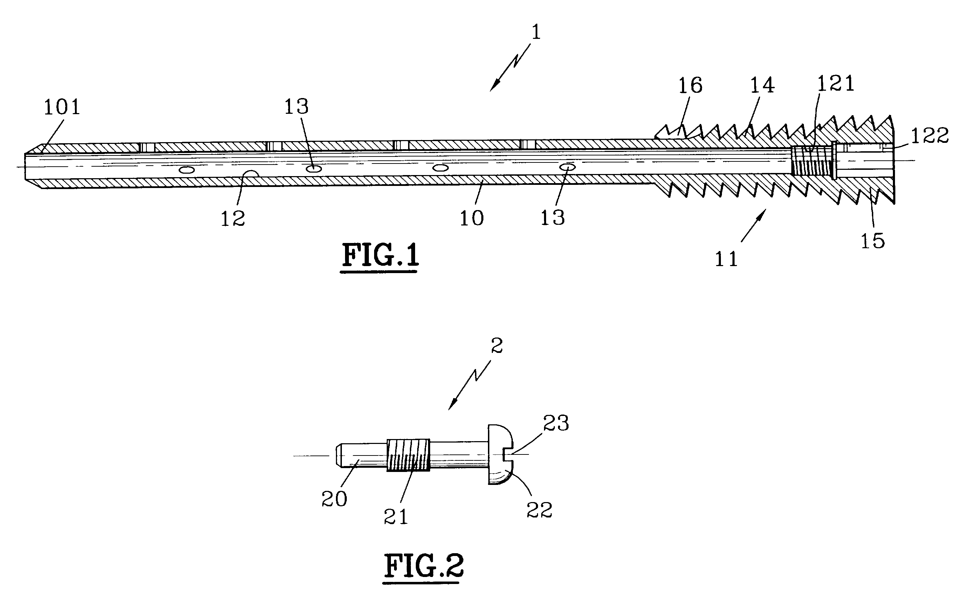

Selon une première réalisation montrée aux figures 1 et 2, un implant d'ostéosynthèse comprend un corps longiligne cylindrique 1 et un bouchon cylindrique 2.

Le corps longiligne cylindrique 1 comprend une portion tubulaire proximale de vascularisation 10 ayant une longueur comprise entre 65 et 105 mm selon l'anatomie du sujet, et une portion d'ancrage distale 11 ayant une longueur de 28 mm. Sur toute la longueur du corps longiligne 1 s'étend un conduit interne 12 ayant un diamètre de 3 mm, à l'exception de deux chambrages 121 et 122 plus larges à son extrémité distale.

Lors de la fabrication, la portion tubulaire 10 a un diamètre extérieur de 5 mm lors de l'usinage du corps longiligne métallique, puis présente un diamètre extérieur final de 5,4 à 5,5 mm après traitement surfacique. Ce traitement consiste à déposer une première couche de titane poreux T40 ayant une épaisseur de 0,2 à 0,3 mm et présentant une porosité comprise entre 20 et 40 %. Puis une substance ostéogénique est déposée en tant que seconde couche sur la première couche. Cette seconde couche a une épaisseur de 0,2 mm environ ; elle peut être un produit pouvant comprendre de la nacre de mollusque, ou bien comprendre du carbonate de calcium CaCO₃ et de la chaux, le carbonate de calcium pouvant être sous la forme au moins en partie de calcite et/ou d'aragonite en plus faible proportion, et la chaux pouvant comprendre de la chaux anhydre CaO et/ou de la chaux hydratée Ca(OH)₂. La seconde couche de revêtement du corps 1 est de préférence déposée par projection thermique au moyen d'une torche à plasma, d'un composé sous forme pulvérulente comprenant l'un des composants suivants : aragonite, calcite, chaux anhydre et chaux hydratée. Cette seconde couche facilite la réossification du noyau de régénérescence autour du corps de l'implant.

L'extrémité proximale du corps est terminée par un tronc de cône 101, ou par un biseau effilé, analogue à celle à l'extrémité d'une seringue, pour faciliter la pénétration du corps dans un trou osseux.

La portion tubulaire comporte plusieurs orifices radiaux 13 de diamètre de 1,8 mm débouchant dans le conduit interne longitudinal 12. Ces orifices sont décalés les uns par rapport aux autres longitudinalement et circonférentiellement le long d'un chemin hélicoïdal coaxial au corps et ayant un pas de 18 mm. Les orifices radiaux 13 sont équirépartis longitudinalement le long de ce chemin hélicoïdal. Selon la réalisation illustrée à la figure 1, trois orifices 13 sont prévus par pas de chemin hélicoïdal et sont équidistribués circonférentiellement d'un pas angulaire de 120° et longitudinalement d'un pas de 6 mm. Les orifices radiaux 13 qui intercommuniquent entre eux à travers le conduit interne longitudinal 12 et l'extrémité proximale de celui-ci contribuent à équilibrer les pressions intracéphaliques dans toute la tête fémorale et jusqu'au trochanter fémoral, après implantation du corps de l'implant. La portion tubulaire perforée 10 constitue un guide de revascularisation par angionèse, et contribue, en addition avec la substance ostéogénique à la surface du corps, à la croissance d'os dur autour du corps 1 de l'implant.

La portion d'ancrage distale 11 est composée de deux tronçons juxtaposés longitudinalement 14 et 15 présentant des filetages autotaraudeurs extérieurs différents.

Le premier tronçon fileté 14 est situé à l'extrémité distale de la portion tubulaire de vascularisation 10 comportant les orifices 13, devant le second tronçon fileté 15. Le premier tronçon fileté présente un diamètre nominal de 8 mm et un pas gros triangulaire, dit pas d'artillerie anti-recul de 2 mm, et un diamètre de fond de filet de 6 mm.

L'extrémité proximale du premier tronçon fileté 14 comporte trois encoches 16 équiréparties circonférentiellement à 120° l'une par rapport à l'autre, comme trois orifices radiaux 13 dans un pas de chemin hélicoïdal précité relativement à la portion tubulaire 10. Les encoches 16 ont un fond affleurant sensiblement la surface externe de la portion tubulaire 10. Selon une réalisation préférée, l'une des parois de chaque encoche est située dans un plan respectif axial au corps 1 et l'autre paroi de l'encoche est située suivant le sens du vissage des filetages 14 et 15 est disposée sensiblement entre 30 et 45° par rapport au plan respectif axial. Les encoches 16 font office de moyen d'amorçage de taraudage dans l'os, lorsque la portion d'ancrage 11 est introduite dans un trou préalablement percé ayant un diamètre de 6,5 mm, sensiblement supérieur au diamètre de fond de filet de 6 mm, ce qui facilite le taraudage de ce perçage par le tronçon fileté 14.

Le second tronçon fileté 15 présente un diamètre nominal de 9 mm avec un pas d'artillerie anti-recul de 2,2 mm et une profondeur de 2 mm environ. Le diamètre nominal et le pas de filetage du second tronçon fileté 15 sont ainsi respectivement plus grands que le diamètre nominal et le pas du premier tronçon fileté 14. La différence de pas entre les premier et second tronçons filetés, égale à 0,2 mm, constitue un moyen anti-dévissage du corps de l'implant pour empêcher le corps de sortir de l'épiphyse, après consolidation de l'os épiphysaire autour et dans les filets de la portion d'ancrage 11 du corps 1 vissé dans l'os épiphysaire.

Comme déjà dit, l'extrémité distale du conduit interne comprend deux chambrages 121 et 122.

Le premier chambrage 121 situé sensiblement à la transition entre les premier et second tronçons filetés 14 et 15 est taraudé à un diamètre intérieur d'écrou de 3,3 mm avec un pas de 0,7 mm. Ce premier chambrage reçoit par vissage un tronçon intermédiaire fileté 21 du bouchon 2.

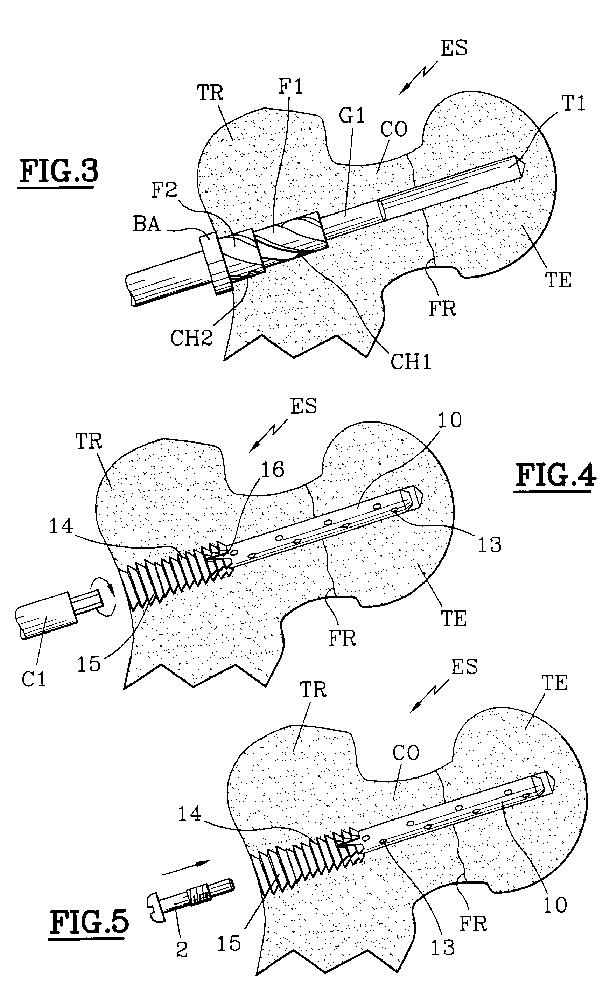

Le second chambrage 122 situé à l'extrémité distale du corps 1 et débouchant vers l'extérieur est sous la forme d'une empreinte femelle à six pans qui circonscrit en section transversale au moins le premier chambrage fileté 121. Les pans du second chambrage 122 ont une largeur de 4 mm et une longueur de 7 mm. Le second chambrage est destiné à recevoir l'extrémité à six pans d'une clé spécifique, comme illustré à la figure 4.

En référence à la figure 2, le bouchon 2 comprend une tige 20 ayant une longueur supérieure à la somme des longueurs des premier et second chambrages 121 et 122 dans le corps 1. La tige 20 est lisse avec un diamètre distal de 2,8 mm, sensiblement inférieur au diamètre du conduit interne 12 du corps 1, à l'exception du tronçon intermédiaire fileté 21. Le tronçon intermédiaire fileté 21 est complémentaire du premier chambrage 121 afin d'être vissé dans celui-ci. L'extrémité distale du bouchon est terminée par une tête plate 22 avec une fente diamétrale 23 pour tournevis. Selon une autre variante, la tête du bouchon est une tête fraisée qui est noyée dans une fraisure ménagée à l'extrémité distale du corps 1 du premier implant, c'est-à-dire à l'extrémité distale du chambrage 122.

Le bouchon 2 obstrue ainsi hermétiquement l'extrémité distale du conduit 12 interne au corps 1 du premier implant et ainsi éviter tout épanchement vasculaire.

Le premier implant selon l'invention est implanté au moyen des quatre outils suivants :
- une broche de 2 mm de diamètre avec une longueur de 200 mm, c'est-à-dire une longueur supérieure à la longueur du corps 1 de l'implant qui varie, en fonction de l'anatomie du patient, entre 93 mm et 133 mm environ ;
- un premier foret creux de diamètre interne égal à 2,1 mm afin d'être guidé par la broche, et de diamètre externe de 5,5 mm afin de percer un trou sensiblement égal au diamètre externe de la portion tubulaire 10 du corps 1 du premier implant ; le foret creux est au moins aussi long que la broche ;
- un outillage de chambrage composé de :
   un premier guide G1 de 5,2 mm de diamètre extérieur et de 28 mm de longueur pour pénétrer dans le trou percé par le premier foret creux ;
   une première fraise de forme F1 de 6,5 mm de diamètre extérieur et de 22 mm de longueur pour percer un premier chambrage à autotarauder par le premier tronçon fileté 14 ; et
   une seconde fraise de forme F2 de 7,5 mm de diamètre extérieur et de 10 mm de longueur pour percer un second chambrage à autotarauder par le second tronçon fileté 15 ; et
- une première clé C1 présentant une extrémité à six pans complémentaire du second chambrage 122 à l'extrémité distale du corps 1 du premier implant, c'est-à-dire présentant des pans ayant une largeur de 4 mm et une longueur d'au moins 7 mm environ.

Les figures 3, 4 et 5 illustrent schématiquement diverses étapes d'usinage dans l'épiphyse supérieure ES d'un fémur afin d'implanter le premier implant 1 selon l'invention. L'épiphyse supérieure du fémur présente une nécrose résultant d'une fracture FR au niveau de la tête fémorale TE.

La broche est d'abord introduite dans l'épiphyse supérieure suivant un axe sensiblement perpendiculaire au trait de la fracture FR et s'étendant de la base du trochanter fémoral TR vers la tête fémorale TE en traversant le col CO et ainsi le foyer de fracture. L'introduction de la broche progresse dans l'os sous le contrôle d'une source de scopie à rayons X. Le trou borgne ainsi réalisé a un diamètre de 2 mm et une profondeur sensiblement supérieure à la longueur du premier implant, bouchon 2 vissé à fond dans le corps 1.

Le trou borgne est élargi en un trou T1 au diamètre de 5,5 mm percé au moyen du premier foret qui glisse autour de la première broche servant de guide longitudinal. Après ce perçage, le premier foret et la première broche sont retirés de l'épiphyse ES.

Les premier et second chambrages sont ensuite fraisés dans le trochanter TR, comme montré à la figure 3. Le premier guide de fraise G1 est enfilé dans le trou T1 qui vient d'être percé, de manière à servir de guide aux deux fraises de forme. Une bague de butée BA à vis de blocage sur le guide G1 limite la profondeur des perçages pour les chambrages. La première fraise de forme F1 élargit le trou T1 en le premier chambrage CH1 au diamètre de 6,5 mm, c'est-à-dire à un diamètre sensiblement supérieur au diamètre de fond de filet du premier tronçon fileté 14 de la portion d'ancrage 11. La longueur du chambrage CH1 est sensiblement supérieure à la longueur du tronçon 14. La seconde fraise de forme F2 élargit ensuite le chambrage CH1 en le second chambrage du diamètre de 7,5 mm, c'est-à-dire à un diamètre sensiblement supérieur au diamètre de fond de filet du second tronçon fileté 15 de la portion d'ancrage 11. La longueur du second chambrage CH2 ainsi débouchant par la face externe du trochanter est sensiblement égale à la longueur du second tronçon fileté 15 à l'extrémité distale du corps d'implant 1.

Après avoir retiré l'outillage de chambrage, le corps d'implant 1 est mis en place dans l'épiphyse supérieure fémorale ES, comme montré à la figure 4. La portion tubulaire 10 est enfilée dans le long trou borgne T1 au moyen d'un impacteur jusqu'à ce que l'extrémité proximale à encoches 16 du premier tronçon fileté 14 de la portion d'ancrage 11 affleure la face externe du trochanter. L'extrémité à six pans de la clé C1 est introduite dans le chambrage à six pans 122 du corps d'implant 1. Une pression est exercée sur la clé C1 de manière à engager l'extrémité distale du premier tronçon fileté 14 dans l'épaulement entre les deux chambrages CH1 et CH2 pratiqués dans le trochanter. La clé C1 est alors utilisée comme tournevis pour visser le corps d'implant 1 dans l'os jusqu'à ce que les premier et second tronçons filetés 14 et 15 du corps d'implant 1 soient logés entièrement dans les deux chambrages respectifs CH1 et CH2 pratiqués dans l'os. Lors de ce vissage, les tronçons filetés 14 et 15 autotaraudent les chambrages CH1 et CH2.

L'implant est ainsi définitivement implanté dans l'éphiphyse supérieure fémorale ES. Grâce à la différence de pas et également de diamètres des tronçons filetés 14 et 15, le corps 1 ne pourra pas être dévissé et reculer en direction distale vers la face externe épiphysaire, après formation osseuse autour du corps. Les orifices 13 et le conduit interne 12 sont en communication de part et d'autre de la fracture FR, dans le col CO et la tête TE et assurent la revascularisation, c'est-à-dire favorisent l'irrigation entre fragment osseux supprimée par la fracture.

La clé C1 est retirée. Comme montré à la figure 5, le bouchon 2 est finalement vissé dans le chambrage fileté 121 du corps 1 jusqu'à ce que la tête 23 soit appliquée contre la face d'extrémité distale du corps et obstrue ainsi l'extrémité distale du conduit 12 interne au corps d'implant 1 qui devient un conduit perforé borgne à embouchure seulement proximale.

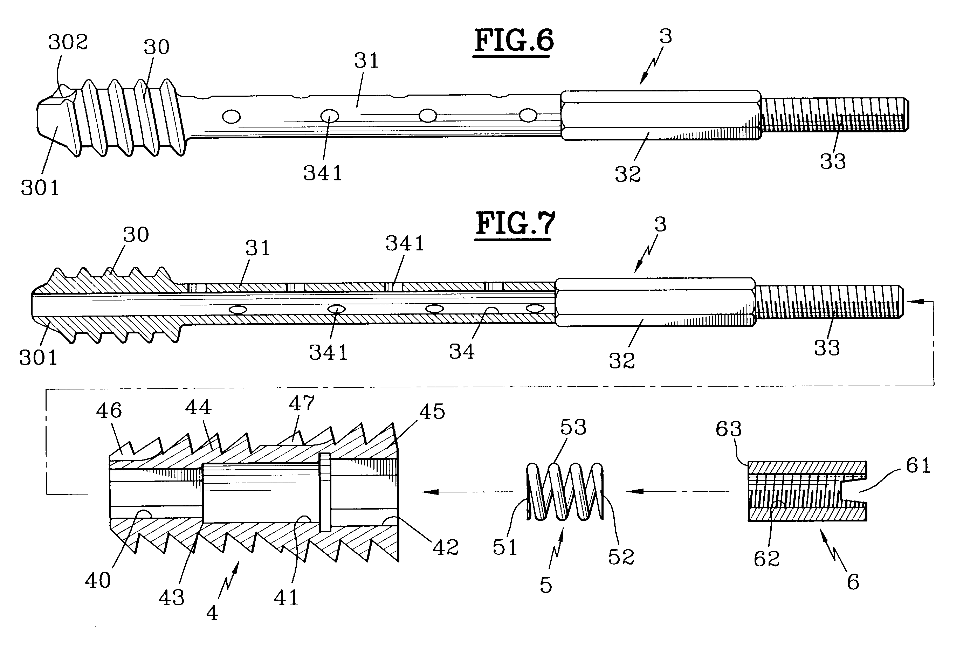

En se référant maintenant aux figures 6 et 7, un second implant d'ostéosynthèse comprend un corps longiligne 3, de forme générale cylindrique sensiblement comme un goujon, un embout distal cylindrique à double filetage externe 4, un ressort hélicoïdal 5 et un bouchon creux taraudé 6.

Le corps longiligne 3 comprend depuis son extrémité proximale vers son extrémité distale, une portion tubulaire proximale 30, filetée et autotaraudeuse, une portion tubulaire lisse perforée 31, une portion de manoeuvre à section hexagonale 32, et enfin une portion distale filetée 33.

Dans la portion proximale 30 et la portion lisse 31 est prévu un conduit de revascularisation interne borgne 34 à embouchure proximale, de diamètre 3 mm. Comme dans la première réalisation, plusieurs orifices radiaux 341 de diamètre 1,8 mm sont pratiqués dans la portion tubulaire lisse 31. Ces orifices sont disposés le long d'un chemin hélicoïdal coaxial au corps 3 et équirépartis longitudinalement avec un pas angulaire de 120°. Les orifices 341 communiquent entre eux à travers le conduit interne longitudinal 34 et avec l'embouchure proximale du conduit interne, celui-ci étant borgne à son extrémité distale au niveau de la portion de manoeuvre 32, comme montré à la figure 7.

Comme dans le corps du premier implant, la portion tubulaire lisse 31 présente un diamètre extérieur de 5,4 à 5,5 mm après dépôts thermiques superficiels d'une première couche de titane poreux et d'une seconde couche d'un produit nacré ou d'un produit comprenant du carbonate de calcium sous la forme de calcite et/ou d'aragonite et de la chaux anhydre et/ou hydratée. La portion tubulaire lisse 31 est prévue en différentes tailles comprises entre 20 et 60 mm pour s'adapter à l'anatomie du patient.

La portion proximale 30 du corps 3 du second implant destinée à être implantée dans la tête fémorale TE présente à la périphérie un filetage sur une longueur de 15 mm avec un diamètre nominal de 8 mm et un diamètre à fond de filet de 6 mm. Ce filetage présente un pas de 3 mm avec un profil spécifique autotaraudeur dit profil de vis pour spongieux ou à os tendre. La portion autotaraudeuse 30 possède une extrémité proximale sous la forme d'un bout tronconique 301 comportant deux encoches diamétralement opposées 302 s'étendant longitudinalement jusqu'à la naissance du filetage autotaraudeur. Les encoches 301 peuvent être similaires aux encoches 16. Le bout tronconique 301 avec les encoches 302 assure l'amorce du filetage autotaraudé dans l'os.

La portion de manoeuvre 32 et la portion filetée 33 du côté de l'extrémité distale du corps 3 ont des longueurs respectives de 25 mm et 10 mm.

Comme montré à la figure 7, l'embout distal 4 comprend un conduit interne composé de premier, second et troisième tronçons ayant des sections transversales de plus en plus grandes, depuis l'extrémité proximale vers l'extrémité distale de l'embout.

Le premier tronçon 40 constitue le tronçon proximal de conduit interne de l'embout 4 et a une section transversale à six pans complémentaire de la section hexagonale du tronçon de manoeuvre 32 du corps 3. Toutefois, la longueur de 10 mm du tronçon d'embout 40 est inférieure à la longueur de 25 mm de la portion à section hexagonale 32 du corps 3. La portion de corps 32 peut ainsi coulisser axialement dans le tronçon d'embout 40 lorsque l'embout 4 est fixé dans le trochanter fémoral TR pour tracter en direction distale le corps 3 ancré par la portion autotaraudeuse proximale 30 dans la tête fémorale TE, comme cela est expliqué dans la suite. La liaison à section polygonale et à coulissement entre la portion 32 et le tronçon 40 permet également de visser la portion autotaraudeuse proximale 30 du corps 3 avec des tronçons de filetage à la périphérie de l'embout 4, comme on le verra dans la suite.

Le tronçon 41 dans l'embout 4 est un tronçon cylindrique lisse situé entre les tronçons proximal et distal 40 et 42. Il a une longueur de 12 mm. Le diamètre de 6,5 mm du tronçon lisse 41 est supérieur à la plus grande largeur de la section hexagonale du tronçon proximal 40 de l'embout mais inférieur à la plus grande largeur de la section hexagonale du tronçon distal 42 de l'embout. Dans le tronçon intermédiaire d'embout 41 est logé complètement le ressort hélicoïdal 5, ayant une longueur au repos de 12 mm, et au moins partiellement le bouchon 6 qui ont des diamètres externes respectifs de 6,3 mm et 6,4 mm. L'extrémité proximale 51 du ressort 5 s'appuie contre un épaulement interne 43 entre les tronçons 40 et 41 sous la poussée exercée par le bouchon 6 appliqué contre l'extrémité distale 52 du ressort 5. La portion de corps 32 peut éventuellement passer à travers le ressort 5.

Les spires du ressort 5 sont imprégnées d'une matière plastique, telle que polyuréthane, de manière à former une gaine 53 autour des spires qui ralentit la détente du ressort après avoir été compressé entre l'épaulement 43 et le bouchon 6. La gaine plastique 53 autour des spires de ressort contribue également à l'atténuation de micro-déplacements et vibrations dont le second implant peut être le siège après implantation.

Le troisième tronçon 42 de l'embout 4 a une section hexagonale et est situé à l'extrémité distale de l'embout. Il présente des pans ayant une largeur de 4 mm et une longueur de 8 mm de manière à recevoir l'extrémité à six pans de la clé précitée C1. Lorsque le tronçon proximal 40 de l'embout 4 est enfilé sur la portion de manoeuvre 32 du corps 3, la portion filetée distale 33 du corps traverse librement les deux autres tronçons de conduit interne 41 et 42 de l'embout. Pour pousser le ressort 5 dans son logement 41, le bouchon 6 est vissé sur la portion filetée distale de corps 33 ayant un diamètre nominal de 4 mm et un pas fin de 0,6 mm.

Comme également montré à la figure 7, la périphérie de l'embout 4 présente un premier tronçon d'ancrage fileté 44 à son extrémité proximale et un second tronçon d'ancrage fileté 45 à son extrémité distale, d'une manière similaire aux premier et second tronçons filetés 14 et 15 composant la portion d'ancrage 11 du corps 1 du premier implant.

Le premier tronçon fileté 44 a un diamètre nominal de 11 mm, un diamètre de fond de filet de 9 mm et un pas d'artillerie anti-recul de 3 mm identique au pas de la portion proximale de corps 30, et s'étend sur une longueur de 20 mm, jusqu'au niveau du tronçon cylindrique lisse 41 du conduit interne d'embout. L'extrémité proximale du premier tronçon autotaraudant 44 comporte trois encoches 46 équiréparties axialement avec un écart angulaire de 120° pour amorcer l'autotaraudage au moyen de ce premier tronçon 44, d'une manière similaire aux encoches 16 à l'extrémité proximale de la portion d'ancrage 11 du corps 1 du premier implant.

Le second tronçon fileté 45 a un diamètre externe de 13 mm et un pas d'artillerie anti-recul de 3,2 mm, et s'étend sur une longueur de 10 mm. L'extrémité proximale du tronçon 45 comporte également trois encoches 47 équiréparties angulairement de 120° pour amorcer l'autotaraudage au moyen de ce second tronçon 45. Les encoches 47 peuvent être décalées radialement par rapport aux encoches 46, par exemple de 60° environ.

La différence des pas, 3,2 - 3 = 0,2 mm, entre les deux tronçons filetés 44 et 45 constitue un moyen d'anti-dévissage de l'embout 4.

Le bouchon taraudé 6 a une longueur qui est inférieure à la somme des longueurs du tronçon lisse intermédiaire 41 et du tronçon à section hexagonale 42 interne à l'embout, et qui est inférieure à la longueur de la portion filetée distale 33 du corps 3 afin que le bouchon 6 soit guidé dans le tronçon lisse 41 et puisse se déplacer axialement de quelques millimètres dans l'embout 4 sous l'action du ressort compressé 5. Le bouchon 6 de diamètre externe de 6,4 mm présente à son extrémité distale une fente 61 grâce à laquelle son alésage taraudé 62 est vissé autour de la portion filetée distale 33 du corps 3 afin que sa face proximale 63 pousse le ressort 5 contre l'épaulement 43 entre le tronçon proximal 40 et le tronçon intermédiaire lisse 41 interne à l'embout. Le bouchon 6 a ainsi un double rôle, la fermeture distale du conduit interne à l'embout, et la mise sous compression du ressort amortissant 5.

Le second implant est implanté au moyen des outils suivants :
- la première broche de 2 mm de diamètre, ayant une longueur supérieure à la longueur du corps 3 du second implant qui peut varier entre 70 mm et 110 mm en fonction de l'anatomie du patient ;
- un second foret creux de diamètre interne égal à 2,1 mm pour être guidé par la broche, et de diamètre externe de 6 mm afin de percer un trou sensiblement égal au plus petit diamètre de fond de filet des filetages du second implant, en l'occurrence celui de la portion filetée proximale 30 du corps 3 ; le second foret creux est au moins aussi long que la broche ;
- un second outillage de chambrage composé de :
   un second guide de 5,7 mm de diamètre extérieur et de 28 mm de longueur pour pénétrer dans le trou percé par le second foret creux ;
   une troisième fraise de forme de 9,5 mm de diamètre extérieur et de 22 mm de longueur pour percer un premier chambrage à autotarauder par le tronçon proximal d'embout 44 ; et
   une quatrième fraise de forme de 11 mm de diamètre extérieur et de 10 mm de longueur pour percer un second chambrage à autotarauder par le tronçon distal d'embout 45 ;
- la première clé spéciale C1 ayant une extrémité à six pans complémentaire du chambrage distal 42 de l'embout, c'est-à-dire présentant des pans ayant une largeur de 4 mm et une longueur d'au moins 8 mm ;
- une seconde clé spéciale ayant un trou d'extrémité cylindrique à taraudage borgne qui est similaire au bouchon 6 pour être vissée sur la portion filetée distale 33 du corps 3 et pénétrer dans les tronçons internes d'embout 41 et 42 de manière à tirer en direction du trochanter TR le corps 3 et particulièrement la tête fémorale TE dans laquelle a été vissée la portion proximale 30 ;
- des brucelles pour la mise en place du ressort 5 ; et
- un tournevis pour visser le bouchon 6 sur le tronçon fileté de corps 33, tout en mettant en compression le ressort 5.

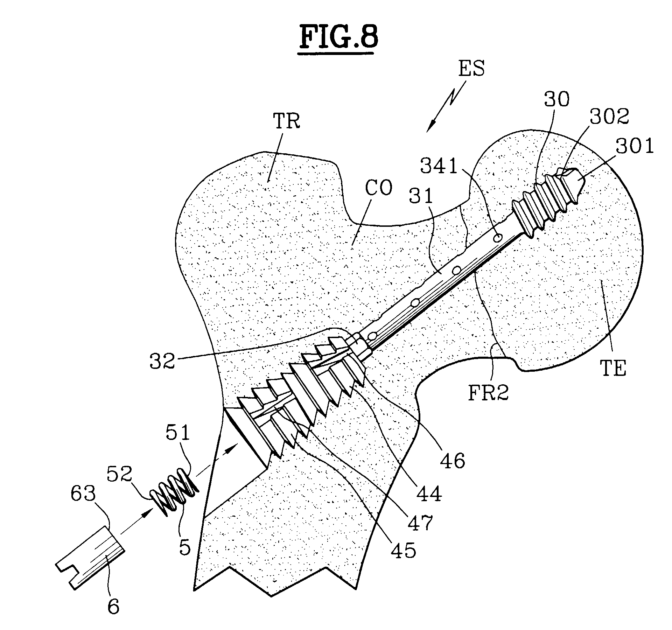

Les diverses étapes de perçage dans l'épiphyse supérieure ES d'un fémur pour implanter le second implant de l'invention sont sensiblement analogues à celles montrées aux figures 3 à 5, en référence à la figure 8. Le second implant est sélectionné de préférence pour des synthèses de fractures FR2 du col du fémur sous capitales, transcervicales, voire basicervicales, à angle de Powles variable.

Comme pour le premier implant, la broche est introduite dans l'épiphyse supérieure ES du fémur, en traversant le trochanter TR, puis le col de fémur CO et enfin partiellement la tête fémorale TE de manière à former un trou borgne sensiblement perpendiculaire à la fracture FR2. La profondeur et la direction de ce trou sont contrôlées par radioscopie, de manière à ce que la longueur ainsi percée soit au moins égale à la longueur hors tout du second implant.

Puis, le second foret est enfilé le long de la broche jusqu'à l'extrémité de celle-ci pour agrandir le trou borgne à un diamètre de 6 mm, qui est sensiblement égal au diamètre de fond de filet de la portion autotaraudeuse proximale 30 du corps 3 du second implant. Le second foret et la broche sont alors retirés de l'épiphyse ES.

Deux chambrages sont ensuite réalisés au moyen du second outillage de chambrage. Le second guide de fraise est introduit dans le trou osseux de 6 mm qui vient d'être percé, et sert de guide aux troisième et quatrième fraises de forme. La troisième fraise de forme perce un premier chambrage de diamètre 9,5 mm sur une profondeur sensiblement supérieure égale à la longueur du tronçon autotaraudeur proximal 44 de l'embout, tandis que la quatrième fraise de forme perce un second chambrage plus large au diamètre de 11 mm sur une profondeur sensiblement supérieure à la longueur du tronçon autotaraudeur distal 45 de l'embout. Les profondeurs des chambrages sont déterminées par une bague de butée, analogue à la bague BA, venant buter en fin de chambrage contre la face externe trochantérienne. Les deux diamètres de fraise de 9,5 et 11 mm sont sensiblement égaux respectivement aux diamètres de fond de filet des tronçons autotaraudeur proximal et distal 44 et 45 de l'embout.

Les outils de chambrage étant retirés, le corps 3 du second implant avec l'embout 4 enfilé sur la portion à section hexagonale 32 est mis en place dans le trou osseux borgne à deux chambrages ainsi réalisé traversant la fracture FR2 dans l'épiphyse supérieure fémorale ES, comme montré à la figure 8. Les encoches proximales 302 et la portion autotaraudeuse proximale 30 du corps 3 mordent l'os et forment dans l'os un taraudage à partir de l'épaulement interne entre le trou long au diamètre de 6 mm et le premier chambrage au diamètre de 9,5 mm. La portion autotaraudeuse proximale 30 poursuit son avance dans le trou osseux borgne. Juste avant d'atteindre le fond de ce trou, une première pression axiale est exercée pour que le tronçon fileté proximal 44 de l'embout autotaraude le premier chambrage, ce tronçon 44 ayant le même pas que la portion de corps 30. Puis une seconde pression sensiblement plus élevée est exercée pour que le tronçon fileté distal 45 de l'embout autotaraude le second chambrage avec un pas plus grand. Les opérations de taraudage sont effectuées grâce à la clé à extrémité hexagonale C1 qui coopère avec le tronçon distal interne 42 de l'embout dont le tronçon interne proximal 40 est engagé autour de la portion à section hexagonale 32 du corps 3.

La portion autotaraudeuse de corps 30 étant ainsi mise en place au fond du trou dans la tête fémorale TE et l'embout 4 étant immobilisé dans le trochanter TR, la seconde clé est vissée sur la portion distale de corps 33. Une traction est exercée avec la seconde clé en direction distale, longitudinalement au corps du second implant, de manière à rapprocher le fragment de tête fémorale TE contre le trochanter TR et ainsi resserrer la fracture.

Après retrait de la seconde clé, le ressort 5 est saisi par les brucelles pour être logé dans le tronçon intermédiaire lisse 41 interne à l'embout, en s'appuyant sur l'épaulement 43.

Le bouchon 6 est vissé au moyen du tournevis autour de la portion filetée distale 33 du corps 3 jusqu'à mettre en compression le ressort 5. Ainsi, le ressort 5 constitue un moyen amortissant propre à pousser en direction proximale l'embout 4 et en direction distale le corps 3, et assure une compression au niveau de la fracture FR2, c'est-à-dire une traction permanente et progressive de la tête fémorale TE dans laquelle le corps 3 est ancré par la portion autotaraudeuse 30, contre le trochanter TR dans lequel l'embout 4 est ancré par les tronçons autotaraudeurs 44 et 45, sans possibilité de dévissage. La compression stabilise la fracture afin de favoriser plus rapidement la consolidation de la fracture.

Une pseudarthrose d'une fracture du col du fémur sous capitale, transcervicale à angle de Powles même oblique peut être traitée en utilisant le second implant selon l'invention. Grâce à la détente latente du ressort 51 poussant l'embout en direction proximale et le corps 3 en direction distale, le second implant met en compression dynamique le foyer de pseudarthrose.

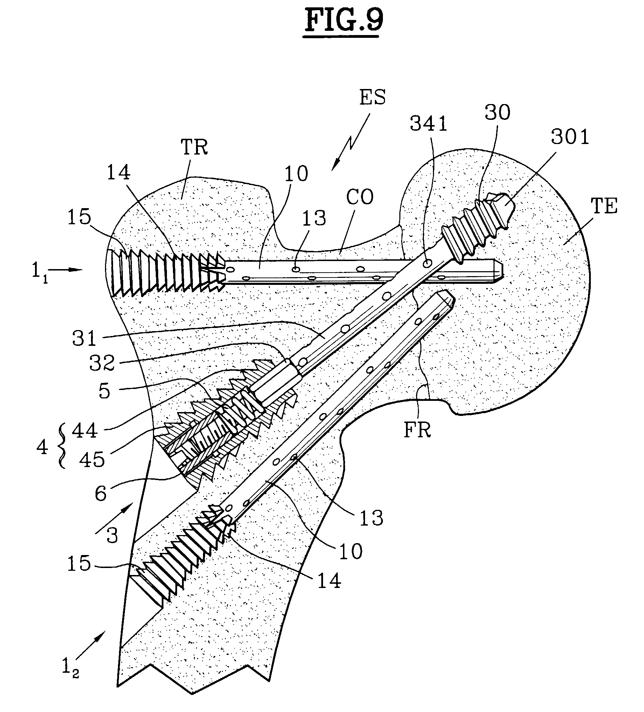

Cette compression peut être complétée par la fonctionnalité d'au moins un premier implant selon l'invention. En référence à la figure 9, deux premiers implants 1₁ et 1₂ sont disposés au-dessus et au-dessous du second implant précédent 3 sensiblement dans le même plan, le plus perpendiculaire possible à celui de la pseudarthrose PS.

La mise en compression dynamique de la pseudarthrose associée aux pouvoirs ostéogéniques des implants par la possibilité d'angiogénèse des portions tubulaires 13 et 31 et du revêtement ostéogénique présent sur leurs surfaces externes permet de traiter ces pseudarthroses sans utilisation de greffe pédiculée, et sans ostéotomie.

Dans ce type d'indication, un montage en triangulation permet l'appui immédiat post-chirurgical. Ce montage est réalisé par la mise en place d'un second implant selon l'invention en compression dynamique axiale dans le col fémoral CO associée à deux premiers implants selon l'invention dont l'un est oblique de bas en haut et l'autre est sensiblement horizontal d'avant en arrière.

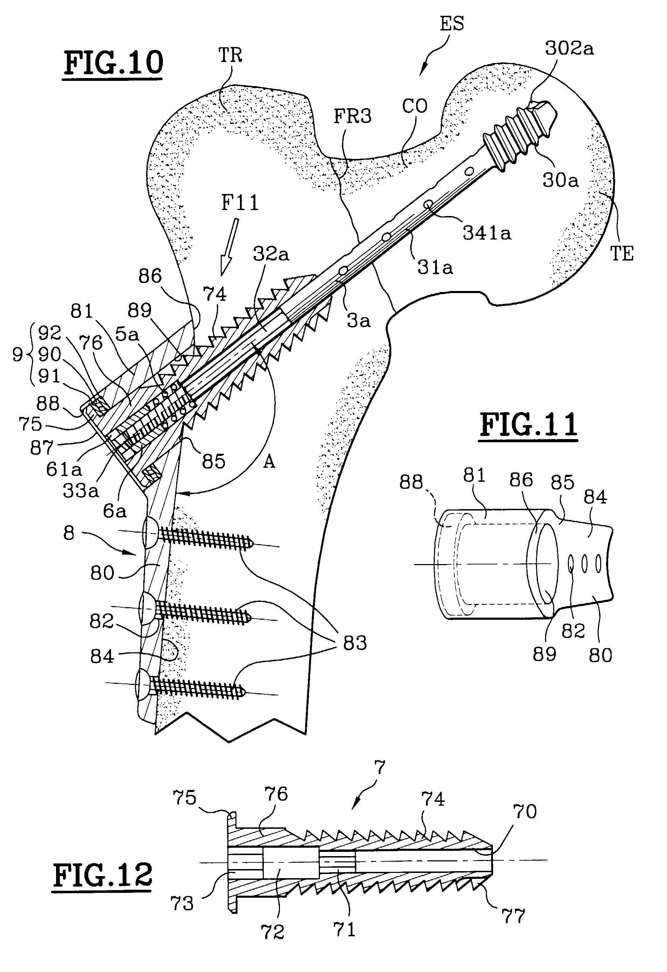

Un troisième implant d'ostéosynthèse selon l'invention est montré aux figures 10, 11 et 12 et comprend un corps longiligne 3a de forme générale cylindrique sensiblement comme un goujon, un embout distal cylindrique 7 à embase et filetage externe, contenant un ressort hélicoïdal 5a et un bouchon creux taraudé 6a, et une plaque diaphysaire 8.

Le corps longiligne 3a est analogue au corps longiligne 3 du second implant montré aux figures 6 et 7, mais est plus long afin que sa portion distale filetée 33a saille de la face externe épiphysaire. Ainsi le corps 3a comprend depuis son extrémité proximale vers son extrémité distale, une portion tubulaire proximale, filetée et autotaraudeuse 30a, une portion tubulaire lisse perforée 31a, une portion de manoeuvre à section hexagonale 32a, et enfin une portion distale filetée 33a. Dans la portion proximale 30a et la portion lisse 31a est ménagé un conduit de revascularisation interne borgne débouchant par une embouchure proximale et par plusieurs orifices radiaux 341a pratiqués dans la portion tubulaire lisse 31a, comme dans les premier et second implants selon l'invention.

Comme dans le corps du premier implant, la portion tubulaire lisse 31a présente un diamètre extérieur de 5,4 à 5,5 mm après dépôts thermiques.

Comme montré à la figure 12, l'embout distal 7 comprend un conduit interne composé de premier, second, troisième et quatrième tronçons 70 à 73 ayant des sections transversales de plus en plus grandes, depuis l'extrémité proximale vers l'extrémité distale de l'embout.

Le premier tronçon 70 est le plus long et constitue le tronçon proximal de conduit interne de l'embout 4. Le tronçon 70 est cylindrique avec une section circulaire sensiblement supérieure à la plus grande largeur de la section hexagonale de la portion de manoeuvre 32a du corps 3a.

Le second tronçon 71 a une section transversale à six pans complémentaire de la section hexagonale de la portion de manoeuvre 32a du corps 3a et s'étend sur une longueur inférieure à la longueur de la portion de manoeuvre 32a du corps 3a afin que la portion de corps 32a puisse coulisser axialement dans le tronçon d'embout 71 lorsque l'embout 7 est fixé dans le trochanter fémoral TR pour tracter en direction distale le corps 3a ancré par la portion autotaraudeuse proximale 30a dans la tête fémorale TE. Comme dans le second implant, la liaison par section polygonale et à coulissement entre la portion de corps 32a et le tronçon 71 permet également de visser dans l'os la portion autotaraudeuse proximale 30a du corps 3a avec un tronçon de filetage à la périphérie de l'embout 7.

Les tronçons 71, 72 et 73 dans l'embout 7 remplissent des fonctions analogues à celles des tronçons 40, 41 et 42 dans l'embout 4.

Le tronçon 72 est un tronçon cylindrique lisse situé entre les tronçons 71 et 73. Le diamètre du tronçon lisse 72 est supérieur à la plus grande largeur de la section hexagonale du tronçon 71 dans l'embout, mais sensiblement égal à la plus petite largeur de la section hexagonale du tronçon distal 73. Dans le tronçon intermédiaire d'embout 72 est logé complètement le ressort hélicoïdal 5a et au moins partiellement le bouchon 6a. L'extrémité proximale du ressort 5a s'appuie contre un épaulement interne entre les tronçons 71 et 72 sous la poussée exercée par le bouchon 6a appliqué contre l'extrémité distale du ressort 5a. Le ressort 5a est de préférence imprégné de matière plastique, comme le ressort 5.

Le troisième tronçon 73 de l'embout 7 a une section hexagonale et est situé à l'extrémité distale de l'embout de manière à recevoir l'extrémité à six pans de la clé précitée C1. Lorsque le tronçon 71 de l'embout 7 est enfilé sur le tronçon de manoeuvre 32a du corps 3a, le tronçon fileté distal 33a du corps 3a traverse librement les deux tronçons de conduit interne 72 et 73 de l'embout. Pour pousser le ressort 5a dans son logement 72, le bouchon 6a est vissé sur la portion filetée distale de corps 33a.

Le bouchon taraudé 6a est analogue au bouchon 6 et peut se déplacer axialement de quelques millimètres dans l'embout 7 sous l'action du ressort compressé 5a, en étant guidé dans le tronçon lisse 72. Le bouchon 6a présente à son extrémité distale une fente 61a afin d'être vissé autour de la portion filetée distale 33a du corps 3a, ce qui comprime le ressort 5a entre la face proximale du ressort 5a et l'épaulement entre le tronçon proximal 72 et le tronçon intermédiaire lisse 71 interne à l'embout 7.

Comme également montré à la figure 12, la périphérie de l'embout 7 présente un long tronçon d'ancrage fileté autotaraudeur proximal 74 s'étendant sur les trois quarts de la longueur de l'embout, et analogue au second tronçon fileté 45 et par conséquent ayant un diamètre et/ou un pas différents de ceux de la portion d'ancrage 30a du corps 3a. Le tronçon d'ancrage fileté 74 présente un traitement surfacique analogue à celui du corps 1.

L'embout 7 présente également à la périphérie une embase circulaire mince distale 75 et un tronçon cylindrique lisse 76 situé entre l'embase et le tronçon d'ancrage 74. Le diamètre du tronçon lisse 76 est plus grand que le diamètre externe du tronçon d'ancrage 74. L'embase 75 et le tronçon 76 qui ne se retrouvent pas dans le second implant, coopèrent avec la plaque diaphysaire 8.

La plaque diaphysaire 8 comprend, en partie inférieure, une portion d'ancrage inférieure longiligne 80 à implanter le long de la face externe diaphysaire du fémur, et en partie supérieure, une portion de guidage et d'appui supérieure 81.

Trois trous 82 sont ménagés dans la portion d'ancrage 80. Les trous 82 s'étendent sensiblement perpendiculairement aux faces de la portion 80 et sont alignés longitudinalement sur celle-ci pour recevoir avec jeu des vis d'ancrage 83.

Comme cela apparaît à la figure 11, la face diaphysaire 84 de la portion d'ancrage a une section transversale concave pour épouser le profil convexe transversal de la diaphyse fémorale.

La portion de guidage et d'appui 81 est un fût cylindrique tronqué qui est oblique par rapport à l'axe longitudinal de la portion d'ancrage 80. L'axe du fût 81 en direction du trochanter TR fait avec la portion d'ancrage 80 un angle A de 120° à 140° environ. La base inclinée proximale 85 du fût 81 est également concave transversalement dans le prolongement de la face diaphysaire 84, et a une extrémité 86 sensiblement convexe longitudinalement de manière à suivre sensiblement le profil incurvé concave dans un plan médio-latéral de l'épiphyse fémorale, à la base du trochanter TR. Le fût cylindrique 81 présente intérieurement dans une base distale 87 perpendiculaire à son axe, un lamage 88 d'un alésage cylindrique lisse axial 89. Le lamage 88 et l'alésage 89 reçoivent à glissement l'embase mince 75 et le tronçon cylindrique distal 76 de l'embout 7 respectivement. La longueur axiale du lamage 88 et de l'alésage 89, c'est-à-dire la plus petite distance séparant les bases 87 et 85 du fût 81, située du côté de la portion d'ancrage 80, est sensiblement supérieure à la longueur axiale de l'embase 75 et du tronçon 76 afin que ceux-ci soient complètement logés dans le fût 81.

Dans le lamage 88 est prévu un moyen 9 pour amortir des micro-déplacements entre l'embout 7 ancré sous le trochanter TR et la plaque diaphysaire 8. Le moyen pour amortir 9 atténue tout effort de levier entre la plaque 8 et l'ensemble de corps 3a et l'embout 7 dû à des microglissements des fragments osseux au niveau du foyer de fracture FR3. Le moyen pour amortir 9 comprend au moins une rondelle 90 en matériau amortissant, tel qu'élastomère ou polyuréthane ou silicone, enfilée sur le tronçon lisse 76. Selon la réalisation illustrée à la figure 10, la rondelle 90 est entretoisée par deux rondelles métalliques 91 et 92 qui sont de préférence thermocollées à la rondelle 90 ; cet ensemble de rondelles 90, 91 et 92 est enfilé autour du tronçon 76 et est enserré à la base distale du tronçon 76, dans le lamage 88, entre l'embase mince distale 75 de l'embout 7 et le fond du lamage 88. Selon une autre variante, l'ensemble de rondelles ne peut comporter qu'une rondelle 90 en matériau amortissant et une seule rondelle métallique 91 ou 92.

Le troisième implant est implanté de préférence pour des fractures sous-trochantériennes FR3. Il est implanté de la manière suivante.

La plaque fémorale 8 est fixée sur la face diaphysaire du fémur, en positionnant la portion de guidage et d'appui 81 sur le coude sous-trochantérien. Les trois vis d'ancrage 83 sont vissées dans le fémur à travers les trous de plaque 82, le cas échéant dans des avant-trous fémoraux préalablement percés, jusqu'à serrer la plaque 8 contre la diaphyse fémorale.

Un guide cylindrique ayant un diamètre interne sensiblement égal à celui d'une broche est introduit à glissement dans l'alésage 89.

La broche plus longue que le corps 3a est introduite, par exemple par rotation manuelle, dans l'épiphyse supérieure ES du fémur, en traversant le guide cylindrique précité, le trochanter TR, la fracture FR3, le col de fémur CO et enfin partiellement la tête fémorale TE de manière à former un trou borgne sensiblement perpendiculaire à la fracture FR3. La profondeur et la direction de ce trou sont contrôlées par radioscopie, de manière à ce que la longueur ainsi percée soit sensiblement égale à la longueur des portions 30a, 31a et 32a du corps 3a.

Puis après avoir retiré le guide cylindrique précité, un foret creux est enfilé le long de la première broche jusqu'à l'extrémité de celle-ci pour agrandir le trou borgne à un diamètre sensiblement égal au diamètre de fond de filet de la portion autotaraudeuse proximale 30a du corps 3a du troisième implant. Le foret et la broche précités sont alors retirés de l'épiphyse ES.

Un guide cylindrique de fraise est introduit dans le long trou osseux qui vient d'être percé, et guide une fraise de forme pour percer un chambrage au diamètre de fond de filet du tronçon autotaraudeur 74 d'embout, sur une profondeur sensiblement égale à la longueur du tronçon 74. La profondeur du chambrage est déterminée par une bague de butée, analogue à la bague BA (figure 3), venant buter en fin de chambrage contre la base externe 87 du fût de plaque 81. Ce chambrage peut ne pas être nécessaire lorsque le diamètre du tronçon d'embout 74 est sensiblement égal à celui de la portion de corps 30a.

Les outils de chambrage étant retirés, le corps 3a du troisième implant avec l'embout 7 enfilé sur la portion à section hexagonale 32a et avec l'ensemble de rondelles 90, 91 et 92 enfilé autour du tronçon lisse d'embout 76 est mis en place dans le trou osseux borgne à chambrage ainsi réalisé traversant la fracture FR3 dans l'épiphyse supérieure fémorale ES, comme montré à la figure 10. Des encoches proximales 302a et le filetage autotaraudeur de la portion proximale 30a du corps 3a mordent l'os et forment dans l'os un taraudage à partir du fond du chambrage. La portion autotaraudeuse proximale 30a poursuit son avance dans le trou osseux borgne. Juste avant que la portion 30a atteigne le fond de ce trou, une première pression axiale est exercée pour que des encoches proximales 77 et le filetage du tronçon autotaraudeur 74 de l'embout 7 mordent et autotaraudent le chambrage osseux. Le tronçon 74 peut avoir le même pas de filetage que la portion de corps 30a ou de préférence un pas plus grand. Les opérations de taraudage sont effectuées grâce à la clé à extrémité hexagonale C1 qui coopère avec le tronçon distal interne 73 de l'embout 7 dont le tronçon interne à section hexagonale 71 est engagé autour de la portion à section hexagonale 32a du corps 3a.

Le tronçon autotaraudeur de corps 30a étant ainsi mis en place au fond du trou osseux dans la tête fémorale TE et l'embout 7 étant immobilisé dans le trochanter TR grâce au tronçon fileté 74, une seconde clé spéciale, analogue à celle à taraudage borgne déjà utilisée pour le second implant, est vissée sur la portion distale de corps 33a. Une traction est exercée avec la seconde clé en direction distale, longitudinalement au corps 3a, de manière à rapprocher le fragment de tête fémorale TE contre le trochanter TR et ainsi resserrer la fracture FR3.

Après retrait de la seconde clé, le ressort 5a est saisi par les brucelles pour être logé dans le tronçon lisse 72 interne à l'embout, en s'appuyant sur l'épaulement entre les tronçons 72 et 71.

Le bouchon 6a est vissé au moyen d'un tournevis autour de la portion filetée distale 33a du corps 3a jusqu'à mettre en compression le ressort 5a. Cette compression assure ainsi au niveau de la fracture FR3, et particulièrement dans les régions trochantérienne et sous-trochantérienne, une traction permanente et progressive de la tête fémorale TE dans laquelle le corps 3a est ancré par la portion autotaraudeuse 30a, contre le trochanter TR dans lequel l'embout 7 est ancré par le tronçon autotaraudeur 74, sans possibilité de dévissage. La compression est répartie le long de la diaphyse fémorale par l'application de la portion d'ancrage de plaque 80 contre la diaphyse, cette répartition étant particulièrement efficace lorsque les efforts exercés dans une fracture sous-trochantérienne FR3 sont élevés. Par rapport à l'assise diaphysaire de l'implant, les effets de levier dûs à des microdéplacements latéraux et des vibrations sont atténués par la rétention de l'extrémité distale 76 de l'embout 7 dans le fût supérieur 81 et le moyen amortissant de rondelles 9 logé dans le lamage de fût 88. Le corps 3a de l'implant est maintenu axialement en lui évitant un balayage intra-céphalique. La répartition de la compression conjuguée à l'atténuation des microdéplacements accentue la consolidation rapide de la fracture.

## Revendications

1. Implant pour ostéosynthèse d'une fracture (FR) entre des premier et second fragments osseux (TE, TR) comprenant un corps longiligne (1 ; 3) ayant une portion autotaraudeuse (11 ; 30) située à l'une des extrémités proximale et distale du corps à implanter respectivement dans les premier et second fragments osseux (TE, TR), caractérisé en ce que dans le corps (1 ; 3) sont prévus des orifices transversaux (13 ; 341) à travers lesquels débouche un conduit longitudinal (12 ; 34) ayant une extrémité distale borgne et une extrémité proximale débouchante (101 ; 301).

2. Implant conforme à la revendication 1, dans lequel le conduit (12) est obstrué par un bouchon (2) à l'extrémité distale du corps (1).

3. Implant conforme à la revendication 2, dans lequel l'extrémité distale du conduit (12) comprend un taraudage (121) dans lequel est vissée une tige filetée (20 ; 21) du bouchon (2).

4. Implant conforme à l'une quelconque des revendications 1 à 3, dans lequel l'extrémité distale du conduit (12) comprend un chambrage (122) débouchant à l'extérieur du corps (1) et ayant une section, de préférence polygonale, pour recevoir une extrémité à section complémentaire d'une clé (Ch) pour visser le corps (1) dans un trou (T1) dans les fragments osseux (TE, TR).

5. Implant conforme à l'une quelconque des revendications 1 à 4, dans lequel la portion autotaraudeuse (11) est située à l'extrémité distale du corps (1) pour être implantée dans le second fragment osseux (TR).

6. Implant conforme à la revendication 5, dans lequel la portion autotaraudeuse (11) est composée de deux tronçons filetés (14 ; 15) ayant des filetages différents, de préférence ayant des pas différents et/ou des diamètres différents.

7. Implant conforme à l'une quelconque des revendications 1 à 6, dans lequel la portion autotaraudeuse (11) comprend un premier tronçon fileté proximal (14) et un second tronçon fileté distal (15) ayant au moins l'un de pas et diamètre nominal respectivement plus grand que l'un respectif de pas et diamètre nominal du premier tronçon fileté (14).

8. Implant conforme à la revendication 1, dans lequel la portion autotaraudeuse (30 ; 30a) est située à l'extrémité proximale du corps (3 ; 3a) pour être implantée dans le premier fragment osseux (TE).

9. Implant conforme à la revendication 1, 5 ou 8, dans lequel l'extrémité distale de la portion autotaraudeuse (11 ; 30 ; 3a) comporte au moins une encoche longitudinale d'amorce de taraudage (16 ; 302 ; 302a).

10. Implant conforme à la revendication 1 ou 8, comprenant à l'extrémité distale du corps (3 ; 3a) un embout (4 ; 7) monté à coulissement autour d'une portion intermédiaire (32 ; 32a) du corps (3 ; 3a) et contenant un moyen amortissant (5 ; 5a) propre à pousser en direction proximale l'embout (4 ; 7) et en direction distale le corps (3 ; 3a).

11. Implant conforme à la revendication 10, dans lequel le moyen amortissant comprend un ressort hélicoïdal (5 ; 5a) de préférence imprégné d'une matière plastique.

12. Implant conforme à la revendication 10, dans lequel le moyen amortissant comprend un ressort hélicoïdal (5 ; 5a) ayant une extrémité proximale (51) appliquée entre un épaulement (43) interne à l'embout (4 ; 7) et une extrémité distale (52) contre laquelle est placé un élément (6 ; 6a) lié au corps (3 ; 3a).

13. Implant conforme à la revendication 12, dans lequel l'élément est un bouchon fileté (6 ; 6a) qui est vissé sur un tronçon fileté (33 ; 33a) situé à l'extrémité distale du corps (3 ; 3a) et qui est logé dans un conduit (41-42 ; 72-73) interne à l'embout (4 ; 7).

14. Implant conforme à la revendication 10 ou 11, dans lequel l'embout (4 ; 7) comprend un conduit interne comportant :
- un chambrage proximal (40 ; 71) ayant une section polygonale pour coulisser sur un tronçon à section polygonale (32 ; 32a) du corps (3 ; 3a), et
- un chambrage intermédiaire (41 ; 72) plus large que le chambrage proximal (40 ; 71) pour recevoir le moyen amortissant (5 ; 5a) appliqué contre un épaulement (43) entre les chambrages proximal et intermédiaire (40, 41 ; 71, 72), et un élément (6 ; 6a) lié à l'extrémité distale (33 ; 33a) du corps (3 ; 3a) et comprimant le moyen amortissant (5 ; 5a) contre l'épaulement.

15. Implant conforme à la revendication 14, dans lequel le conduit interne dans l'embout (4 ; 7) comporte un chambrage distal (42 ; 73) ayant une section, de préférence polygonale, pour recevoir une extrémité complémentaire de clé (C1) pour visser le corps (3 ; 3a) et l'embout (4 ; 7) respectivement dans les premier et second fragments osseux (TE, TR).

16. Implant conforme à l'une quelconque des revendications 10 à 15, dans lequel la périphérie de l'embout (4) comprend un tronçon autotaraudeur (44), de préférence ayant un pas sensiblement égal à celui de la portion autotaraudeuse (30) du corps (3).

17. Implant conforme à l'une quelconque des revendications 10 à 16 lorsqu'elle dépend de la revendication 8, dans lequel la périphérie de l'embout (4 ; 7) comprend un tronçon autotaraudeur (45 ; 74) ayant un filet différent de celui de la portion autotaraudeuse (30 ; 30a) située à l'extrémité proximale du corps (3 ; 3a).

18. Implant conforme à l'une quelconque des revendications 10 à 15, dans lequel la périphérie de l'embout (4) comprend à son extrémité proximale un premier tronçon autotaraudeur (44) et à son extrémité distale un second tronçon fileté (45) ayant au moins l'un des pas et diamètre nominal respectivement plus grand que le pas et le diamètre nominal du premier tronçon autotaraudeur (44).

19. Implant conforme à l'une quelconque des revendications 10 à 18, comprenant une plaque (8) ayant une portion d'ancrage (80) pour être fixée contre la diaphyse du second fragment osseux (TR), et une portion cylindrique (81) qui est traversée à glissement par l'embout (7) et contre laquelle une embase distale (75) de l'embout (7) est appliquée.

20. Embout conforme à la revendication 19, dans lequel la portion cylindrique (81) est oblique par rapport à la portion d'ancrage (80).

21. Implant conforme à la revendication 19 ou 20, comprenant un moyen (9) situé entre l'embase distale (75) de l'embout (7) et la portion cylindrique (81) de la plaque (8) pour amortir des déplacements relatifs entre l'embout (7) et la plaque (8).

22. Implant conforme à la revendication 21, dans lequel le moyen pour amortir (9) comprend une rondelle en matériau amortissant (90) qui est de préférence en contact avec une ou deux rondelles métalliques (91, 92).

23. Implant conforme à l'une quelconque des revendications 19 à 22, dans lequel la plaque (80) a une face (84) à appliquer contre la diaphyse du second fragment (TR), qui est concave transversalement.

24. Implant conforme à l'une quelconque des revendications 19 à 23, dans lequel la portion cylindrique (81) de la plaque (8) est tronquée et présente une base qui est inclinée (85) par rapport à l'axe de la portion cylindrique et concave transversalement et sensiblement convexe longitudinalement.
